(19) Europäisches Patentamt
European Patent Office
Office européen des brevets

(11) **EP 3 141 187 B1**

(12) **FASCICULE DE BREVET EUROPEEN**

(45) Date de publication et mention
de la délivrance du brevet:
**30.08.2017 Bulletin 2017/35**

(51) Int Cl.:
*A61B 6/03* *(2006.01)* *A61B 6/00* *(2006.01)*

(21) Numéro de dépôt: **16186349.3**

(22) Date de dépôt: **30.08.2016**

(54) **MIRE ET PROCÉDÉ DE CALIBRATION D'UN DISPOSITIF D'IMAGERIE PAR RAYONS X**

MESSLATTE UND KALIBRIERUNGSVERFAHREN EINER BILDGEBENDEN VORRICHTUNG
DURCH RÖNTGENSTRAHLEN

TEST PATTERN AND METHOD FOR CALIBRATING AN X-RAY IMAGING DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **11.09.2015 FR 1501892**

(43) Date de publication de la demande:
**15.03.2017 Bulletin 2017/11**

(73) Titulaire: **Thales**
**92400 Courbevoie (FR)**

(72) Inventeurs:
• **BERNARD, Guillaume**
**38340 VOREPPE (FR)**
• **MURIENNE, Albert**
**38000 GRENOBLE (FR)**

(74) Mandataire: **Derval, Estelle et al
Marks & Clerk France
Conseils en Propriete Industrielle
Immeuble Visium
22, avenue Aristide Briand
94117 Arcueil Cedex (FR)**

(56) Documents cités:
**US-A- 6 044 132      US-A1- 2005 094 771
US-A1- 2005 147 206      US-A1- 2010 046 718
US-A1- 2014 114 173**

**Description**

La présente invention concerne un dispositif de calibration géométrique d'un dispositif d'imagerie à rayons X. Elle se rapporte plus particulièrement aux dispositifs d'imagerie dont le détecteur est bidimensionnel, c'est à dire, sensiblement plan. Le dispositif d'imagerie comprend classiquement une source de rayons X et un détecteur bidimensionnel. Les rayons X sont atténués par un corps à imager. L'énergie restante est captée par le détecteur et est convertie en signaux de façon à produire une image radiologique. L'image radiologique est une image bidimensionnelle (2D) (ou projection) d'un objet déployé dans un espace à trois dimensions (3D) interposé entre la source de rayons X et le détecteur bidimensionnel. Classiquement, la source de rayons X et le détecteur sont installés sur un dispositif d'entrainement permettant de les déplacer par rapport au corps à imager de façon à réaliser plusieurs projections du corps sous des angles de vue différents. Les projections sont alors utilisées pour reconstruire une image tridimensionnelle du corps imagé. Lors des prises de vue, le détecteur et la source doivent normalement suivre des trajectoires prédéterminées, par exemple des trajectoires circulaires. Or, du fait des tolérances du dispositif de déplacement, il existe des erreurs entre la trajectoire réellement suivie par chacun de ces éléments et la trajectoire idéale qu'il devrait suivre. Autrement dit, il existe des erreurs entre les positions idéales de la source et/ou du détecteur par rapport à l'objet et les positions qu'ils occupent réellement. La reconstruction de l'image 3D à partir des projections 2D de l'objet tient compte des positions relatives entre l'objet, le détecteur et la source à chaque prise de vue. Il est donc fondamental de tenir compte des erreurs de positionnement définies précédemment afin d'obtenir des images 3D présentant une bonne résolution et une bonne précision. Autrement dit, il est fondamental de connaitre les caractéristiques géométriques du dispositif d'imagerie par rayons X lors de l'acquisition de chaque projection 2D à partir desquelles l'image 3D est construite. Le dispositif de calibration tenant compte de ces erreurs de positionnement est classiquement basé sur l'utilisation d'une mire géométrique de calibration connue dans l'espace 3D. La mire de calibration comprend un objet connu comprenant un certain nombre de marqueurs (marqueurs radio-opaques) dont les positions dans l'espace sont connues par construction par des coordonnées mesurées par rapport à un repère propre à cet objet. On acquiert une projection de la mire dans les conditions géométriques que l'on souhaite calibrer, c'est-à-dire selon le point de vue que l'on souhaite calibrer. Ce point de vue est défini par les positions du détecteur, de la source et de la mire et par leurs orientations relatives. On reconnaît, sur la projection de la mire, les projections des marqueurs. Les positions des projections des marqueurs dans la projection de la mire ainsi que la connaissance de leurs positions respectives sur la mire permettent de déterminer les caractéristiques géométriques du dispositif d'imagerie lors de l'acquisition de l'image selon le point de vue adopté. Les caractéristiques géométriques du dispositif d'imagerie par rayons X lors de l'acquisition d'une image selon le point de vue adopté sont définies par une matrice de projection 4*3 qui permet de mettre en correspondance chaque point de l'objet dans l'espace 3D (par rapport au référentiel terrestre) avec sa projection sur un détecteur plan 2D (par rapport à un référentiel lié au détecteur). Ces caractéristiques géométriques sont avantageusement utilisées lors de l'étape de reconstructions 3D.

Les mires classiques comprennent un objet cylindrique transparent aux rayons X muni à sa surface de billes absorbant les rayons X formant les marqueurs. Les billes sont classiquement espacées le long d'une hélice. Or, ce type d'agencement ne permet pas d'intégrer un nombre important de billes sur l'objet cylindrique ce qui nuit à la précision de la calibration et par conséquent de la reconstruction de l'image 3D.

La mire est classiquement munie d'une bille de référence présentant une taille différente des autres billes. Les positions des autres billes relativement à cette bille de référence sont connues. C'est l'identification et le positionnement de la projection de cette bille de référence sur une image 2D, la connaissance des positions des autres billes par rapport à la bille de référence et les positions des projections des autres billes par rapport à la projection de la bille de référence qui permettent de mettre en correspondance les positions des projections de toutes les billes de l'image et leurs positions dans l'espace 3D et ainsi d'obtenir la matrice de projection au moyen d'algorithmes adéquats. La présence et l'identification de la projection de la bille de référence sur la projection de l'objet est donc fondamentale. Or, si la projection de cette bille se superpose à celle d'une autre bille sur une image, ce qui peut arriver pour certains angles de vue, si la projection de cette bille n'apparait pas sur l'image, ce qui peut être le cas si l'image de la mire est tronquée, ou bien si l'image est de mauvaise qualité, on peut alors attribuer une projection détectée sur une image 2D à une mauvaise bille de l'espace 3D ou se retrouver dans l'impossibilité d'attribuer les projections aux billes correspondantes. La calibration est alors impossible ou erronée. Autrement dit, ce type de mire conduit à des procédés de calibration manquant de robustesse par rapport aux identifications erronées de la projection de la bille de référence (fausses détections). Par ailleurs, il peut s'avérer complexe et coûteux d'identifier la projection de la bille de référence parmi un ensemble de projections de billes avec un bon niveau de confiance.

Du brevet américain US 6,715,918, on connait une mire qui ne nécessite pas l'identification d'une bille de référence. Cette mire comprend un corps cylindrique et des marqueurs arrangés le long d'une hélice. Les marqueurs comprennent deux types de marqueurs différenciés par leurs propriétés géométriques, par exemple leurs tailles et/ou par leurs formes respectives. A chacun des deux types de marqueurs physiques est associée une valeur binaire. Les valeurs affectées à une séquence de marqueurs successifs forment alors un code binaire. Les marqueurs du premier et du deuxième type sont agencés en hélice de façon que lorsque l'on prend un nombre prédéterminé de marqueurs successifs, le code binaire obtenu n'apparait qu'une seule fois le long de l'hélice et ce quelque soit le sens de lecture. Or, cette mire présente un certain nombre d'inconvénients. En effet, les marqueurs étant différenciés par leurs propriétés géométriques, et notamment par leurs tailles, plus un marqueur est proche de la source de rayons X, plus sa projection est grande. Par conséquent, on ne peut pas garantir, même en prévoyant des marqueurs de tailles très différentes, que les projections des marqueurs des deux types soient différenciables aisément pour tous les angles de prise de vue. Il y a donc un risque non nul de les confondre sur les projections obtenues ce qui conduit à attribuer une image d'un marqueur d'un type à un marqueur d'un autre type, et limite la fiabilité de la calibration.

La fiabilité de la calibration est également limitée du fait de la disposition des marqueurs en hélice. Lorsqu'une source de lumière éclaire la mire selon un axe central perpendiculaire à l'axe du cylindre, les distances entre les projections des marqueurs éclairés par les bords du faisceau émis par la source sont très faibles et les images de ces marqueurs peuvent être superposées. Cela rend plus difficile et moins fiable l'attribution d'une projection d'un marqueur à un marqueur donné de la mire du fait de la complexité de la forme en hélice et limite la précision de la calibration. Une solution donnée dans le brevet US 6, 715,918 est d'exclure les bords du cylindre de la zone de projection de façon à éviter les superpositions des projections des marqueurs. Toutefois, cette solution, en excluant certains marqueurs des projections, conduit à une limitation de la précision. Une autre solution donnée dans le brevet US 6, 715,918 est de prévoir un algorithme de détection de marqueurs permettant d'exclure de la calibration les projections des marqueurs situées sur les bords du cylindre par rapport à l'axe central. Toutefois, cela conduit à augmenter la complexité de la calibration et n'est pas fiable à 100%.

Un but de l'invention est de remédier à au moins un des inconvénients précités.

A cet effet l'invention a pour objet une mire de calibration destinée à calibrer géométriquement un dispositif d'imagerie par rayons X destiné à générer des images tridimensionnelles d'un objet par reconstruction à partir de projections bidimensionnelles dudit objet, la mire de calibration comprenant un support volumique muni de marqueurs à absorption radiologique contrastée par rapport au support volumique, les marqueurs étant répartis selon une figure tridimensionnelle. Les marqueurs sont répartis en sous-ensembles de marqueurs répartis selon des droites respectives sensiblement parallèles de façon que des séquences de birapports puissent être construites à partir des sous-ensembles de marqueurs respectifs, chaque séquence de birapports comprenant un unique birapport pour chaque quadruplet de marqueurs dans lequel les marqueurs sont ordonnés selon un ordre dépendant des numéros d'ordre des marqueurs respectifs le long de la droite selon laquelle ils sont alignés selon un premier sens prédéfini, ledit ordre étant commun à tous les birapports, et lorsqu'un sous-ensemble de marqueurs comprend au moins cinq marqueurs, l'ordre des birapports dans les séquences de birapports respectives suivant une règle prédéfinie commune à toutes les séquences de birapports. Avantageusement, les séquences de birapports sont toutes différentes.

La mire de calibration selon l'invention permet de mettre en oeuvre des procédés robustes, simples et fiables de calibration. En effet, la mise en correspondance entre une projection d'un marqueur et le marqueur associé sur la mire est réalisée à partir des positions de projections de marqueurs détectées sur une image (projection de la mire). Or, la détection de projections de marqueurs de la mire selon l'invention est fiable. En effet, elle est indépendante de la taille et de la forme des marqueurs. Cette détection peut par ailleurs être obtenue par des algorithmes de détection très simples (de type détecteur de blobs circulaires). Il n'est pas nécessaire que ces algorithmes permettent de différencier des projections de marqueurs de géométries différentes.

Par ailleurs, la mise en correspondance des marqueurs et de leurs projections respectives sur une image ou projection 2D de la mire n'est pas réalisée directement. Elle est réalisée en mettant en correspondance des chaines de birapports uniques formées par les projections des marqueurs d'une part et par les marqueurs de la mire d'autre part. Le risque de faire une erreur lors de la mise en correspondance est donc beaucoup plus faible que lors d'une mise en correspondance réalisée projection par projection. La mire selon l'invention permet par conséquent de mettre en oeuvre des procédés de détection robustes.

Lors d'une prise d'image par rayon X, l'opération géométrique mise en oeuvre est une projection conique (projection centrale) du corps imagé. L'image d'une droite est une droite et les birapports entre les distances sont conservés par projection centrale. Les risques de superposition des images des différents marqueurs en bordure du faisceau dont le rayon central est sensiblement perpendiculaire à un axe perpendiculaire aux alignements de marqueurs est donc limité ce qui permet de réaliser une calibration fiable. Cette propriété permet de profiter des projections de la totalité des marqueurs pour réaliser la calibration ce qui permet de réaliser une calibration précise. La répartition des marqueurs selon des droites parallèles joue également en faveur de la précision et de fiabilité de la calibration car le nombre de marqueurs pouvant être intégrés dans un cylindre est important.

La répartition des marqueurs et par conséquent des projections des marqueurs selon des lignes droites permet d'extraire facilement et avec une bonne fiabilité les informations codées formées par les projections des marqueurs, ici des suites de birapports, en utilisant un simple algorithme de détection d'alignements dans les projections 2D. Elle permet de faciliter la mise en correspondance, avec un bon degré de certitude, de portions de lignes droites projetées avec les lignes droites correspondantes sur la mire. Par ailleurs, la disposition connue des marqueurs selon des droites et par conséquent la disposition à priori des projections de marqueurs selon des droites peut permettre de corriger des petites erreurs de positionnement des projections de certains marqueurs qui s'éloignent de la droite attendue et ainsi de réaliser une calibration précise.

La mire de calibration comprend avantageusement un des caractéristiques suivantes ou plusieurs des caractéristiques suivantes :

- tous les marqueurs présentent sensiblement la même taille et sensiblement la même forme ;
- les droites sont des génératrices dites remarquables d'un cylindre ;
- la répartition des droites selon lesquelles les sous-ensembles de marqueurs sont alignés est choisie de façon que, pour toutes les conditions de projection dans lesquelles on acquiert des images lors de la calibration aucune projection de marqueur ne chevauche une autre projection de marqueur et/ou de façon que si des projections de marqueurs issues de deux sous-ensembles de marqueurs se chevauchent, les projections des marqueurs des autres sous-ensembles de marqueurs ne se chevauchent pas ;
- les sous-ensembles de marqueurs sont répartis selon des segments droites remarquables respectifs parallèles à un axe , les segments de droite étant de même longueur et présentant les même coordonnées selon ledit axe, chaque segment de droite hébergeant un premier nombre m entier de sites, susceptibles d'être occupés par un marqueur, deux sites consécutifs quelconques pris le long dudit segment remarquable étant espacés d'une distance prédéterminée appelée pas, chaque site étant affecté d'une première valeur ou respectivement d'une deuxième valeur selon qu'il est occupé par un marqueur ou non, les marqueurs étant répartis de façon que les valeurs attribuées à un deuxième nombre entier n, au plus égal à m, de sites consécutifs quelconques pris selon un sens donné le long des segments de droites respectifs forment des codes binaires respectifs composés de n bits, chaque code binaire composé de n bits formé selon ledit sens étant unique ;
- le deuxième nombre m est supérieur au premier nombre n ;
- les marqueurs sont répartis sur la mire de façon que, pour un nombre de génératrices et de sites par génératrice connus et pour des taux d'occupation connus des sites génératrices respectives, une différence entre des codes binaires formés par les valeurs prises par les m sites consécutifs hébergés par les génératrices remarquables respectives selon le sens prédéfini est maximale, les codes binaires sont des portions d'une suite obtenue au moyen d'un LFSR de n bits, m étant inférieur ou égal à n ;
- l'ordre des birapports dans chaque séquence de birapports construite à partir d'un sous-ensemble de marqueurs alignés selon une droite, est défini de la façon suivante :

Pour des marqueurs notés Ag portent les numéros d'ordre g = 1 à N, avec N nombre entier, le long de la droite selon un deuxième sens,

Pour i allant de 1 à N-3 faire :
(-Pour j allant de i+1 à N-2 faire :
(-Pour k allant de j+1 à N-1 faire :
(-Pour l allant de k+1 à N faire :
(-insérer le birapport suivant dans la séquence, le birapport suivant étant un birapport calculé à partir des marqueurs $A_{g=i}$, $A_{g=j}$, $A_{g=k}$, $A_{g=l}$,
- l=l+1)
- k=k+1)
- j=j+1),
-i=i+1) ;

- l'ordre des marqueurs dans chaque quadruplet de marqueurs est l'ordre des marqueurs le long de la droite selon laquelle ils sont alignés selon le premier sens.

L'invention se rapporte également à un procédé de détermination de caractéristiques géométriques d'un dispositif d'imagerie par rayons X, destiné à réaliser des images tridimensionnelles d'un objet par reconstruction à partir d'images bidimensionnelles dudit objet, le procédé utilisant une mire de calibration selon l'invention, le procédé selon l'invention comprend les étapes suivantes :

- Placer la mire de calibration dans une zone de projection entre une source de rayons X et un détecteur de rayons X,
- acquérir au moins une projection de la mire de calibration selon au moins une géométrie du dispositif d'imagerie définie par des positions de la source, de la mire et du détecteur et de leurs orientations relatives,

Et, pour chaque projection de la mire :

  ◦ Détecter les projections de marqueurs dans la projection,
  ◦ Déterminer les positions des projections de marqueurs dans la projection,
  ◦ Détecter des alignements de projections des marqueurs selon des droites respectives appelées droites images,

Pour chaque alignement de projections de marqueurs :

  ◦ Former une séquence dite séquence image de birapports à partir des projections de marqueurs formant ledit alignement, la séquence image comprenant un unique birapport par quadruplet de projections de marqueurs dans lequel les projections de marqueurs sont ordonnées selon l'ordre prédéfini dépendant des numéros d'ordre des projections marqueurs respectives le long de la droite image correspondante selon le premier sens prédéfini, et , lorsqu'un ensemble de projections de marqueurs comprend au moins cinq projections marqueurs, l'ordre des birapports dans la séquences image suivant la règle prédéfinie le long de la droite image correspondante,
  ◦ Pour chaque projection de marqueur formant ledit alignement détectée, identifier le marqueur dont elle est issue en attribuant une séquence de birapports formée à partir des projections de marqueur dudit alignement à une partie d'une séquence de birapports formée par des marqueurs de la mire comprenant le même nombre de birapports que la séquence image,
  ◦ Déterminer des caractéristiques géométriques du dispositif d'imagerie lors de l'acquisition de la projection de la mire à partir des positions des projections de marqueurs détectées et des positions des marqueurs correspondants respectifs.

Le procédé présente avantageusement au moins une des caractéristiques ci-dessous :

- l'étape de détection des alignements est réalisée en appliquant une transformée de Hough aux positions des projections de marqueurs détectées lors de l'étape de détection des projections des marqueurs ;
- les codes binaires sont des portions d'une suite obtenue au moyen d'un LFSR de n bits.

D'autres caractéristiques et avantages de l'invention apparaîtront à la lecture de la description détaillée qui suit, faite à titre d'exemple non limitatif et en référence aux dessins annexés dans lesquels :

- la figure 1 représente schématiquement un dispositif d'imagerie par rayons X dans une configuration dans laquelle on acquiert une image de la mire de calibration selon l'invention ;
- la figure 2 représente schématiquement des marqueurs alignés selon une droite remarquable et des projections de marqueurs selon une autre droite,
- les figures 3a et 3b représentent schématiquement une génératrice remarquable d'une mire selon l'invention et les sites qu'elle héberge (figure 2a) ainsi que les marqueurs disposés sur cette génératrice (figure 2b),
- la figure 4 représente un schéma synoptique des étapes du procédé selon l'invention.

D'une figure à l'autre, les mêmes éléments sont repérés par les mêmes références.

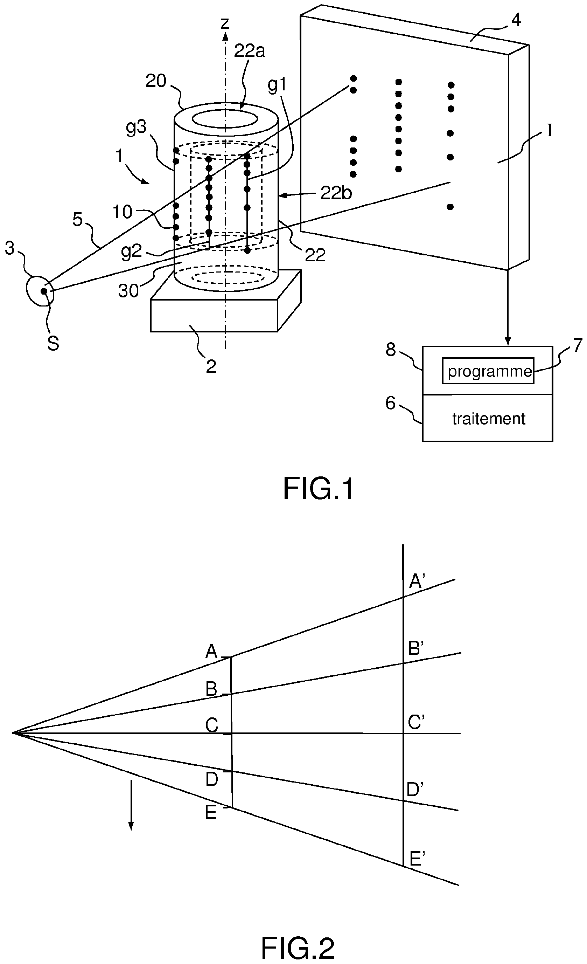

La figure 1 représente un dispositif d'imagerie par rayons X. Ce dispositif d'imagerie comprend une source de rayons X 3 comprenant un foyer S et un détecteur plan 4. Le dispositif d'imagerie comprend avantageusement un dispositif d'entraînement non représenté permettant de déplacer la source 3 et le détecteur plan 4 par rapport à un support 2 destiné à recevoir un objet à imager.

La mire de calibration 1 selon l'invention est ainsi interposée entre une source de rayons X 3 et le détecteur plan 4. Elle est fixe par rapport au support 2. Le dispositif de calibration comprend également un dispositif de traitement 6 qui est configuré pour mettre en oeuvre au moins un procédé de traitement contenu dans un programme 7 stocké dans une mémoire programme 8. La mire 1 comprend des marqueurs 10. Avantageusement, on stocke dans la mémoire 8 un programme contenant un procédé de traitement permettant de calculer des caractéristiques géométriques du dispositif d'imagerie à partir de l'association entre des projections des marqueurs de la mire sur des projections 2D respectives de la mire et les positions, sur la mire, des marqueurs qui ont généré ces projections. La projection I de la mire sur le détecteur plan est représentée schématiquement sur la figure 1.

Le faisceau de rayons X 5 émis par la source 3 depuis le foyer S présente la forme d'un cône qui s'élargit depuis la source 3 jusqu'au détecteur plan 4. L'opération géométrique intervenant dans la production d'une image est donc une projection conique (ou projection centrale) de la mire 1 déployé dans l'espace 3D sur le plan formé par le détecteur plan.

La mire de calibration 1 comprend un support volumique 20 et un ensemble de marqueurs 10 portés par le support volumique 20 et répartis selon une figure tridimensionnelle. Les marqueurs 10 sont fixes par rapport au support volumique 20. Ils sont par exemple fixés sur la surface du support (comme sur la figure 1) ou incrustés dans le support volumique 20. Par support volumique on entend un support présentant une forme tridimensionnelle. Le support volumique est par exemple un cylindre creux (comme sur la figure 1) ou plein. Le cylindre présente avantageusement une symétrie de révolution autour d'un axe z. Il présente une courbe directrice circulaire et une droite génératrice perpendiculaire au plan contenant le cercle directeur (courbe directrice).

Les marqueurs 10 sont à absorption radiologique contrastée par rapport au support volumique 20. De cette façon la détermination des positions des images (ou projections) des marqueurs respectifs sur l'image de manière fiable est possible.

De préférence, le support 20 est transparent aux rayons X. Il est par exemple réalisé en plexiglas en PVC ou en polycarbonate.

Les marqueurs absorbent les rayons X. Ils sont par exemple réalisés en acier inoxydable ou en tout autre matériau similaire absorbant plus les rayons X que le support 20.

La forme et la taille des marqueurs doivent être connues avec précision de manière à faciliter leur détection et le positionnement des barycentres de leurs images respectives, c'est-à-dire de leurs projections respectives, sur les images 2D acquises.

Les marqueurs 10 présentent avantageusement la même taille, la même forme et avantageusement les mêmes caractéristiques d'absorption aux rayons X. Cela permet de faciliter la détection et le positionnement des projections ou barycentres des projections des marqueurs sur les images 2D. Les marqueurs présentent avantageusement une forme sphérique. Cette forme facilite la détection et le positionnement des projections des marqueurs sur les images 2D puisque la projection conique d'une sphère est toujours une ellipse proche d'un disque donc facilement positionnable. En variante, les marqueurs présentent une autre forme. Ils présentent avantageusement une symétrie de révolution autour d'axes de symétrie de révolution respectifs. Ils sont avantageusement positionnés de façon que leurs axes de symétrie de révolution respectifs soient sensiblement parallèles à l'axe z ou avantageusement sensiblement parallèles aux génératrices remarquables qui seront décrites ultérieurement. Il peut s'agir de cylindres ou de barres présentant des formes allongés selon des axes respectifs. Avantageusement, les marqueurs sont homogènes de façon à présenter dans tout leur volume les mêmes caractéristiques d'absorption aux rayons X ce qui facilite leur détection et leur positionnement.

Dans une variante, l'ensemble de marqueurs comprend des marqueurs dont au moins une des caractéristiques précités diffère.

Les positions des marqueurs 10 respectifs sur le support volumique 20 sont connues avec une précision suffisante permettant de réaliser la calibration géométrique avec la précision souhaitée. Par exemple, la position des marqueurs par rapport au support volumique 20 est connue avec une précision de l'ordre de 20 micromètres.

La répartition des marqueurs sur la mire selon l'invention présente plusieurs caractéristiques.

Les marqueurs sont répartis en sous-ensembles de marqueurs. Les marqueurs de chaque sous-ensemble de marqueurs sont répartis en ligne droite. Autrement dit, les marqueurs d'un même sous-ensemble sont disposés sur une ligne droite et espacés le long de cette ligne droite. Les droites selon lesquelles s'étendent les marqueurs des sous-ensembles respectifs sont parallèles.

Une séquence de birapports aussi appelés rapports anharmoniques peut être construite à partir des marqueurs de chaque sous-ensemble de marqueurs. Chaque birapport est calculé à partir de distances entre les marqueurs d'un quadruplet de marqueurs d'un même sous-ensemble de marqueurs.

Chaque birapport est calculé à partir d'un quadruplet de marqueurs dans lequel les marqueurs sont ordonnés selon un ordre prédéterminé dépendant des numéros d'ordre des marqueurs respectifs le long de la droite selon un premier sens prédéfini.

Cet ordre est commun à tous les birapports, c'est-à-dire à tous les quadruplets de marqueurs Autrement dit, on construit un unique birapport par quadruplet de marqueurs successifs.

Lorsqu'un sous-ensemble de marqueurs comprend au moins cinq marqueurs, la séquence de birapports correspondante comprend plusieurs birapports. L'ordre des birapports dans chaque séquence suit une règle prédéfinie commune à toutes les séquences de birapports. Cette règle est définie en fonction des rangs, c'est-à-dire des numéros d'ordre, des marqueurs formant les quadruplets de marqueurs respectifs d'un sous-ensemble, à partir desquels sont calculés les birapports respectifs, le long de la droite correspondante selon un deuxième sens prédéterminé. Le rang ou numéro d'ordre d'un marqueur d'un sous-ensemble est son rang dans une suite, formée par les marqueurs du sous-ensemble correspondant, dans laquelle les marqueurs du sous-ensemble sont classés par ordre de succession des marqueurs le long de la droite correspondante selon le deuxième sens.

Le deuxième sens est avantageusement le premier sens.

Par exemple, pour quatre marqueurs tels que représentés sur la figure 2 alignés selon une droite d. Chaque birapport est calculé selon le sens de la flèche. On appelle r, la fonction birapport. Le birapport r calculé pour les marqueurs A, B, C et D pris dans cet ordre le long de la droite correspondante dans le sens de la flèche est donné par l'équation suivante :

$$r(A, B, C, D) = \frac{\dfrac{\overline{CA}}{\overline{CB}}}{\dfrac{\overline{DA}}{\overline{DB}}}$$

La séquence de birapports construite à partir des marqueurs A, B, C, D et E est par exemple la séquence suivante :

- r1 = r(A,B,C,D)
- suivi du birapport r2 = r(A,B, C, E)
- suivi du birapport r3= r(A,C,D,E)
- suivi du birapport r4 = r(A,C, D,E)
- suivi du birapport r5 = r(B,C,D, E).

Prenons un sous-ensembles de N marqueurs alignés selon une droite. N est un nombre entier. Ces marqueurs notés $A_g$ portent les numéros d'ordre g, avec g= 1 à N, le long de la droite selon le deuxième sens.

Avantageusement, l'ordre des birapports dans la séquence est défini de la façon suivante :

Pour i allant de 1 à N-3 faire :

    (-Pour j allant de i+1 à N-2 faire :

        (-Pour k allant de j+1 à N-1 faire :

            (-Pour l allant de k+1 à N faire :

                (-insérer le birapport suivant dans la séquence, le birapport suivant étant un birapport calculé à partir des marqueurs $A_{g=i}$, $A_{g=j}$, $A_{g=k}$, $A_{g=l}$,

                - l=l+1)

            - k=k+1)

        - j=j+1),

    -i=i+1).

Cette règle de construction de la séquence permet de garantir l'unicité des séquences de birapports construites à partir d'un ensemble de marqueurs, en particulier lorsque la répartition des marqueurs est réalisée comme dans le mode de réalisation particulier décrit plus loin dans la description dans lequel les valeurs affectées à des sites régulièrement espacés selon un segment de droite, pris selon un sens donné le long du segment de droite, forment des codes binaires uniques de n bits.

Chaque birapport est calculé à partir d'un quadruplet de marqueurs dans lequel les marqueurs sont ordonnés selon un ordre de succession prédéterminé dépendant des numéros d'ordre des marqueurs respectifs le long de la droite correspondante selon un premier sens prédéfini.

Avantageusement, pour le calcul des birapports, l'ordre des marqueurs dans chaque quadruplet de marqueurs est l'ordre des marqueurs le long de la droite selon laquelle ils sont alignés selon le premier sens prédéfini. En variante, l'ordre des marqueurs dans chaque quadruplet de marqueurs est l'ordre selon le sens contraire au premier sens prédéfini.

Chaque séquence de birapports est unique sur la mire. Autrement dit, les séquences de birapports formés à partir des sous-ensembles de marqueurs respectifs de la mire sont toutes différentes. Autrement dit, les séquences de birapports sont différentes les unes des autres. Elles sont distinctes.

Il faut noter que la détermination d'une configuration géométrique d'acquisition du dispositif d'imagerie par rayons X de manière fiable est possible, en imageant la mire dans cette configuration, lorsque l'on sait associer chaque projection d'un marqueur sur l'image 2D avec le marqueur de la mire qui a généré cette image. La mire selon l'invention permet cette association car chaque séquence de birapports est unique. En effet, par la projection conique intervenant dans la génération d'une image radiologique, une droite est une droite et les birapports sont conservés. Par conséquent, comme visible sur la figure 2, la projection de chaque sous-ensemble de marqueurs A, B, D, C, E est un alignement d'un sous-ensemble de projections de marqueurs A', B', C', D', E'. La séquence de birapports formée à partir des projections de marqueurs A', B', C', D', E' du sous-ensemble de projections de marqueurs est identique à la séquence de birapports formée à partir des marqueurs du sous-ensemble de marqueurs correspondant A, B, C, D, E si ces séquences sont construites en suivant le même ordre de calcul de chacun des birapports et la même règle d'ordonnancement des birapports respectifs. Par conséquent, une fois ces séquences construites, on peut associer la projection d'un marqueur au marqueur correspondant en associant une séquence d'un sous-ensemble de marqueurs à une séquence de projections de marqueurs. Il n'est pas nécessaire de prévoir un marqueur de référence.

La mire selon l'invention permet de réaliser une calibration de façon simple et fiable. En effet, il suffit de détecter les projections des marqueurs, de les positionner, de détecter des alignements de projections de marqueurs c'est-à-dire de former les sous-ensembles respectifs de projections de marqueurs, de construire les séquences de birapports à partir des projections de marqueurs formant les alignements (ou sous-ensembles) respectifs et d'associer les séquences de birapports respectives à des séquences respectives de birapports de sous-ensembles de marqueurs de la mire. Avantageusement, la séquence de birapports formée à partir d'un sous-ensemble de marqueurs attribuée à celle formée à partir d'un sous-ensemble de projections de marqueurs est celle qui, parmi l'ensemble des séquences de birapports formées à partir de tous les sous-ensembles de marqueurs respectifs, présente une différence minimale avec la séquence de birapports formée à partir d'un sous-ensemble de projections de marqueurs. Or, l'opération de détection d'alignements est une opération bien connue et maitrisée par l'homme du métier. Cette opération est simple et fiable. Il en est de même pour le calcul des distances inter-marqueurs et inter-projections de marqueurs.

Par ailleurs, la solution selon l'invention permet, du fait de la disposition en ligne droite, de corriger des positionnements erronés. En effet, étant donné que l'attribution d'un marqueur à une projection d'un marqueur est réalisée de façon collective, si le positionnement de la projection d'un marqueur est erroné, l'attribution de cette projection au marqueur correspondant est réalisée avec une meilleure probabilité.

La construction des séquences de birapports des projections de marqueurs est indépendante de la géométrie des marqueurs. Elle dépend uniquement des distances séparant les projections de différents marqueurs ce qui limite les risques d'identifier des séquences erronées quelque soit l'angle de la prise de vue.

Avantageusement, les sous-ensembles de marqueurs sont répartis selon des segments de droites respectifs parallèles à l'axe z présentant des longueurs respectives identiques et des coordonnées respectives identiques selon l'axe z.

Avantageusement, les segments de droites sont des génératrices remarquables d'un cylindre. Avantageusement, le cylindre est à symétrie de révolution autour de l'axe z. Il s'agit par exemple du cylindre 22.

Cet exemple est représenté sur les figues 1 et 3a et 3b.

Sur la figure 1, les marqueurs 10 sont répartis selon une pluralité de génératrices remarquables g1, g2, g3 d'un cylindre 22 espacées le long de la courbe directrice du cylindre 30. Sur la réalisation non limitative de la figure 1, le cylindre 22 est inclus dans le cylindre formé par le corps volumique 20, il présente le même axe z et la surface formée par les droites génératrices du cylindre 22 est une portion de la surface extérieure 22b du cylindre formé par le corps volumique 20. En variante, le cylindre est tout autre cylindre solidaire du corps volumique 20 et de préférence coaxial avec le corps volumique 20.

Les génératrices remarquables g1, g2, g3 selon lesquelles sont répartis les marqueurs 10 sont espacées deux à deux, le long de la courbe directrice, d'une distance fixe ou variable. Autrement dit, elles sont espacées deux à deux d'un angle fixe ou variable autour de l'axe z du cercle directeur dans le cas d'un cylindre de révolution. Plusieurs marqueurs sont positionnés sur chaque génératrice remarquable g1, g2, g3 et espacés deux à deux le long de ladite génératrice remarquable. Autrement dit, les marqueurs sont agencés en lignes droites.

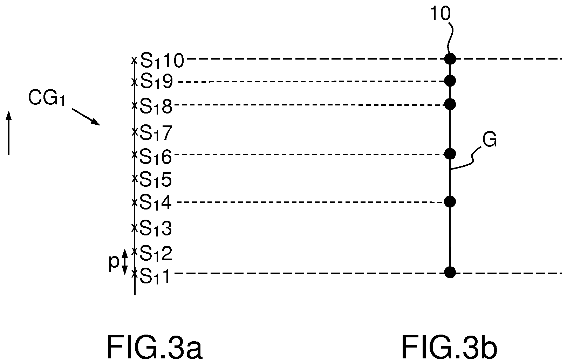

Sur les figures 3a et 3b, on a représenté un exemple préféré d'agencement des marqueurs selon la génératrice remarquable g1. Sur la figure 3a, on a représenté par des croix, des sites $S_ij$ avec j = 1 à 10 que la génératrice remarquable gi=1 comprend. Sur la figure 3b, on a représenté les marqueurs positionnés le long de cette première génératrice remarquable. Chaque génératrice remarquable héberge un premier nombre m entier de sites $S_ij$, j = 1 à m (avec m = 10 sur l'exemple non limitatif de la figure 1) susceptibles d'être occupés par un marqueur 10. Ces sites $S_ij$ sont espacés d'une distance fixe appelée pas p le long de ladite génératrice g1. Autrement dit, deux sites consécutifs quelconques pris le long de la génératrice remarquable, selon un sens donné représenté par la flèche, sont séparés d'une distance p fixe. Sur la figure 3b, on remarque que les sites $S_1 1$, $S_1 4$, $S_1 6$, et $S_1 8$ à $S_1 10$ sont occupés par un marqueur 10 et

que les sites $S_12$, $S_13$, $S_15$ et $S7$ sont libres. Les marqueurs consécutifs 10 selon la droite génératrice g1 sont donc séparés par une distance égale à un multiple du pas p.

Chaque site est affecté d'une première valeur (=1) ou respectivement d'une deuxième valeur (=0) selon qu'il est occupé par un marqueur 10 ou non respectivement. Sur l'exemple des figures 3a et 3b, les sites $S_11$, $S_14$, $S_16$, et $S_18$ à $S_110$ sont affectés de la valeur 1 car ils sont occupés par un marqueur et les sites $S2$, $S_13$, $S_15$ et $S_i7$ sont affectés de la valeur 0 car ils ne sont pas occupés par un marqueur 10.

Les valeurs attribuées à un deuxième nombre entier n de sites consécutifs quelconques pris le long d'une génératrice remarquable g1, selon un sens donné (représenté par la flèche) de la génératrice remarquable, forment une unité d'information binaire composée de n bits (ou mot). Par unité d'information de n bits, on entend également un mot, un code binaire ou une séquence binaire de n bits. Sur la réalisation des figures 3a et 3b, les valeurs attribuées aux 10 sites compris dans la génératrice g1 forment une séquence de code comprenant 3 mots de codes de 8 bits chacun, c'est-à-dire trois unités d'information composées de 8 bits chacune. Ces unités d'information I1, I2, I3 sont formées des valeurs attribuées aux sites S1 à S8, S2 à S9 et respectivement S3 à S10. Ces codes binaires sont les suivants :

- I1 : 1 0010101
- I2 : 00101011
- I3 : 01010111

La séquence de code formée par les valeurs attribuées aux 10 sites de la génératrice g1 est la suivante : 1001010111.

Chaque unité d'information de n bits prise sur une génératrice remarquable dans le sens donné selon ladite droite, ledit sens étant commun à toutes les génératrices remarquables, doit apparaitre une seule fois sur la mire. Autrement dit, chaque unité d'information de n bits prise selon le sens donné est unique. Elle apparait une seule fois sur l'ensemble des génératrices remarquables. Le sens prédéfini de lecture est le sens de lecture défini parallèlement à l'axe du cylindre. Avantageusement, le sens est le premier sens et/ou le deuxième sens.

En plaçant les marqueurs de cette façon sur la mire on obtient de manière simple et rapide des sous-ensembles de marqueurs permettant de former des séquences de birapports (telles que définies précédemment) respectives uniques sur la mire. Par ailleurs, une telle mire est facile à fabriquer, le réglage des positions des marqueurs respectifs étant simple. Avantageusement, chaque séquence de birapports comprenant plus d'un birapport est formée de portions de la séquence de birapports uniques sur la mire. Les portions d'une séquence sont lues dans le même sens que la séquence correspondante. Les portions de la séquence présentent une longueur W inférieure à celle de la séquence. La longueur W des portions de séquence correspond au nombre de birapports dans les portions de séquence. Par exemple, sur l'exemple des figures 3a et 3b, la séquence B1, B2, B3, B4 est formée de deux séquences uniques de longueur trois : B1, B2, B3 et B2, B3, B4 ou de trois séquences uniques de longueur deux : B1, B2 et B2, B3 et B3, B4.

Cela permet d'augmenter la robustesse de la calibration géométrique, réalisée à partir de la mire selon l'invention, à la troncature de la projection de la mire. En effet, si tous les marqueurs ne sont pas présents sur la projection, il est possible de faire correspondre, avec un bon degré de certitude, les projections de marqueur présentes avec les marqueurs correspondants en associant des séquences de projections de marqueurs de longueur W avec des portions de séquence de marqueurs correspondantes.

Dans le mode de réalisation des figures 3a et 3b, cela se répercute de la façon suivante. Chaque chaîne de marqueurs disposés selon une génératrice du cylindre présentant une longueur L (exprimée en mètres) représente une séquence de code de longueur m bits.

Avantageusement, m est supérieur à n. Autrement dit, la longueur L des génératrices est supérieure ou égale à n*p. La probabilité d'imager une chaîne de marqueurs de longueur n*p est plus importante que celle d'imager une longueur m*p. Or, l'unité d'information de n bits est unique sur la mire, connue et sa position est connue sur la mire ce qui permet de mettre en correspondance de façon fiable les projections des marqueurs présents dans cette chaîne avec les marqueurs de cette chaîne. En variante m est égal à n.

Sur la réalisation de la figure 3b, la génératrice remarquable g1 héberge m = 10 sites et les unités d'information sont codées sur n= 8 bits. Dans les modes de réalisation préférés de l'invention, ces nombres m et n sont bien supérieurs.

Typiquement, on prévoit des unités d'informations de n= 16 bits et des chaînes de marqueurs représentant des séquences de code d'une longueur m compris entre 24 et 40 bits.

Avantageusement, les chaînes de marqueurs représentent des séquences de code binaire comprenant une proportion de 1 comprise entre 7 et 18. Avantageusement, la mire comprend au moins 6 sous-ensembles de marqueurs.

Avantageusement, chaque séquence de birapports ou portion de séquence de birapports est unique dans les deux sens. Cela permet de réduire les risques de mise en correspondance erronées. Toutefois, cette condition n'est pas essentielle car ce risque est limité du fait que les unités d'informations sont réalisées selon des droites dont la géométrie est invariante et prédictible par projection lors d'une prise d'image par rayons X.

Chaque unité d'information est avantageusement unique dans le sens prédéfini et dans un autre sens opposé audit sens.

Avantageusement, la répartition des génératrices remarquables g1, g2, g3 le long de la courbe directrice 30, ou plus généralement des droites selon lesquelles sont réalisés les alignements, est choisie de façon que, pour toutes les conditions de projection dans lesquelles on acquiert des images lors de la calibration, c'est-à-dire pour toutes les géométries d'acquisition d'image utilisées lors de la calibration (définies par les positions de la source, du détecteur et de la mire et leurs orientations respectives), dans lesquelles le rayon central du faisceau émis par la source 3 est incliné par rapport à l'axe z d'au plus 45°, et dans lesquelles le détecteur est sensiblement perpendiculaire au rayon central, aucune projection de marqueur ne chevauche une autre projection de marqueur et/ou de façon que si des projections de marqueurs issues de deux sous-ensembles de marqueurs se chevauchent, les projections des marqueurs des autres sous-ensembles de marqueurs ne se chevauchent pas. Par rayon central, on entend le rayon qui se trouve au centre du faisceau émis par la source 3 de rayons X, c'est-à-dire l'axe de symétrie du faisceau émis par la source 3. Cela permet de faciliter la discrimination des projections des marqueurs.

La fabrication de la mire de calibration selon l'invention comprend une étape de placement des marqueurs le long de l'ensemble des droites ou de l'ensemble des génératrices remarquables selon une répartition telle que les séquences de birapports respectives soient uniques. Cette répartition peut être obtenue par itérations successives d'une étape de placement des marqueurs, d'une étape de construction des séquences de birapports correspondantes et de vérification de l'unicité des séquences.

A cet effet, l'agencement est avantageusement réalisé de façon que les valeurs attribuées à n sites consécutifs quelconques pris le long de l'une quelconque des génératrices remarquables, selon le sens de la génératrice remarquable, forment une unité d'information composée de n bits, l'unité d'information étant unique dans la mire selon ledit sens. La répartition des marqueurs peut être obtenue de plusieurs manières. Elle est par exemple obtenue par itérations successives d'une étape de placement des marqueurs, d'une étape de construction des unités d'information et de vérification de l'unicité des unités d'informations ou bien au moyen d'un registre à décalage à rétroaction linéaire de taille n, chaque unité d'information de n bits étant une portion, c'est-à-dire une séquence de n bits, d'une suite d'une longueur inférieure à $2^n$ engendrée par un registre à décalage à rétroaction linéaire (LFRS en référence à l'expression anglo-saxonne : « linear feedback shift register ») à n bits. L'utilisation d'un LFRS est bien adaptée à une mise en oeuvre matérielle et est simple à mettre en oeuvre. Par ailleurs, l'utilisation de séquences de n bits d'une suite engendrée par un LFSR à n bits permet de garantir l'obtention de séquences uniques, c'est-à-dire d'unités d'information, uniques sur la mire. En effet, un LFRS codé sur 16 bits garantit de générer une suite comprenant 65535 séries de 16 bits toutes différentes, les séries de 16 bits étant les états consécutifs de la LFSR à 16 bits.

Prenons le cas où chaque génératrice remarquable héberge un nombre m supérieur à n sites. Par exemple, lorsque n = 16, alors m est compris entre 24 et 40. La répartition des marqueurs sur les sites de la génératrice forme par exemple un code correspondant à une suite de m bits obtenue au moyen d'un LFSR à 16 bits. Cette répartition est simple car elle évite une étape de vérification de l'unicité des m-n séquences de code de 16 bits formés par les valeurs attribuées aux sites selon leur occupation ou non par un marqueur puisque cette unicité est garantie par les propriétés mathématiques du LFSR. Par ailleurs, elle garantit l'unicité du code dans les deux sens.

Avantageusement, pour améliorer la fiabilité de la calibration géométrique réalisée au moyen d'une mire selon l'invention, les marqueurs sont répartis sur la mire de façon que pour un nombre de génératrices et de sites par génératrice connus et pour des taux d'occupation connus des sites des génératrices respectives, une différence entre des codes binaires formés par les valeurs prises par les m sites consécutifs hébergés par les génératrices remarquables respectives selon le sens prédéfini soit maximale. Autrement dit, les séquences choisies dans la suite générée par la suite obtenue par le LFSR à 16 bits sont celles qui présentent une différence maximale. La différence calculée entre deux codes binaires est par exemple la différence entre le nombre de bits de valeur 1 du résultat du ou exclusif des deux codes binaires.

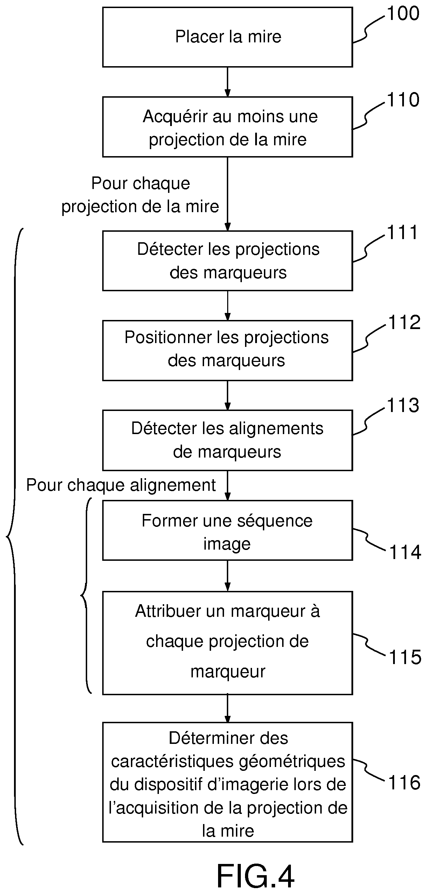

L'invention a également pour objet un procédé de détermination des caractéristiques géométriques du dispositif d'imagerie par rayons X lors de l'acquisition d'au moins une image. Le procédé utilise une mire de calibration selon l'invention. Le procédé selon l'invention comprend les étapes suivantes, représentées sur la figure 4 :

- Placer 100 la mire de calibration dans une zone de projection entre la source de rayons X et le détecteur de rayons X,
- acquérir 110 au moins une projection de la mire selon au moins une géométrie du dispositif d'imagerie ; il est possible d'acquérir plusieurs projections selon des géométries différentes par exemple selon plusieurs positions angulaires d'une trajectoire circulaire de la source et du détecteur autour de l'axe z du cylindre,

Et, pour au moins une projection acquise :

- Détecter 111 les projections de marqueurs dans la projection, cette étape est mise en oeuvre par une méthode classique, comme par exemple de façon non limitative, par une méthode dite de détection de blobs signifiant large objet binaire, en référence à l'expression anglo saxonne « binary large object », ou par un procédé de reconnaissance de forme, notamment de détection d'ellipses,

- Déterminer 112 les positions des projections de marqueurs ; cette étape peut être réalisée lors de l'étape 111 ;
- Détecter 113, sur la projection, des alignements de projections des marqueurs selon des droites respectives di, appelées droites image ; cette étape est par exemple réalisée par une méthode classique, par exemple du type régression linéaire ou au moyen d'une transformée de Hough ; cette étape permet d'identifier les droites selon lesquels sont alignées les projections des marqueurs et d'identifier les projections de marqueurs alignées selon ces droites respectives;
- Pour chaque alignement i détecté :

  ◦ Former 114 une séquence dite séquence image de birapports à partir des projections de marqueurs formant ledit alignement, la séquence image comprenant un unique birapport par quadruplet de projections de marqueurs dans lequel les projections de marqueurs sont ordonnées selon l'ordre prédéfini dépendant des numéros d'ordre des projections de marqueurs respectives le long de la droite image correspondante selon le premier sens prédéfini et, lorsqu'un ensemble de projections de marqueurs comprend au moins cinq projections marqueurs, l'ordre des birapports dans la séquences image suivant la règle prédéfinie le long de la droite image correspondante (cette règle est par conséquent commune à tous les alignements, elle est définie en fonction des rangs ou numéros d'ordre des projections marqueurs respectives formant les quadruplets de projections marqueurs générant les birapports respectifs le long de la droite image correspondante selon le deuxième sens prédéterminé, cette règle est la même que la règle de constructions des séquences de birapports),
  ◦ Pour chaque projection de marqueur formant ledit alignement détectée, identifier 115 le marqueur dont elle est issue en attribuant une séquence de birapports formée à partir des projections de marqueur dudit alignement à une partie d'une séquence de birapports formée par des marqueurs de la mire comprenant le même nombre de birapports que la séquence image,

- Déterminer 116 des caractéristiques géométriques du dispositif d'imagerie lors de l'acquisition de la projection de la mire à partir des positions des projections de marqueurs détectées et des positions des marqueurs correspondants respectifs. Cette étape consiste notamment à calculer une matrice de projection de l'espace 2D vers l'espace 3D. Elle est par exemple réalisée en utilisant l'algorithme de Faugeras-Toscani ou de Levenberg-Marquardt.

Lors de l'étape 115, le marqueur dont est issue une projection est identifié en associant une séquence de birapports formée à partir des projections de marqueur dudit alignement à une partie d'une séquence de birapports formée par des marqueurs de la mire comprenant le même nombre de birapports que la séquence image.

L'étape d'identification 115 des marqueurs dont sont issues chacune des projections de marqueurs qui ont été détectées consiste avantageusement à identifier quelle partie de séquence de marqueurs de la mire présente une différence minimale avec la séquence image ou est identique à la séquence image. La différence minimisée est par exemple l'écart quadratique moyen de la différence des deux séquences de birapports ou l'écart quadratique moyen d'une différence pondérée des deux séquences de birapports pour compenser le fait que les birapports sont tous différents alors que l'erreur, elle, est sensiblement identique.

Avantageusement, l'étape de détection des alignements est réalisée en appliquant une transformée de Hough aux seules positions des projections de marqueurs détectés lors de l'étape de détection des projections des marqueurs. Cela revient à appliquer une transformée de Hough à une image binaire comprenant un fond uniforme et des pixels contrastés par rapport au fond uniforme dont les positions respectives sont les positions des projections de marqueurs qui ont été détectées lors de l'étape 112. Cela permet de détecter les droites selon lesquelles sont alignés les pixels et d'éviter que la transformée de Hough ne détecte les contours des projections des marqueurs. Les risques d'erreur sont donc limités. Par ailleurs, cela simplifie l'algorithme de Hough et limite les temps de calcul.

## Revendications

1. Mire de calibration (1) destinée à calibrer géométriquement un dispositif d'imagerie par rayons X destiné à générer des images tridimensionnelles d'un objet par reconstruction à partir de projections bidimensionnelles dudit objet, la mire de calibration (1) comprenant un support volumique (20) muni de marqueurs (10) à absorption radiologique contrastée par rapport au support volumique (20), les marqueurs (10) étant répartis selon une figure tridimensionnelle, **caractérisé en ce que** les marqueurs (10) sont répartis en sous-ensembles de marqueurs répartis selon des droites respectives sensiblement parallèles de façon que des séquences de birapports puissent être construites à partir des sous-ensembles de marqueurs respectifs, chaque séquence de birapports comprenant un unique birapport pour chaque quadruplet de marqueurs dans lequel les marqueurs sont ordonnés selon un ordre dépendant des numéros d'ordre des marqueurs respectifs le long de la droite selon laquelle ils sont alignés selon un premier sens prédéfini, ledit ordre étant commun à tous les birapports, et lorsqu'un sous-ensemble de marqueurs comprend au moins cinq marqueurs, l'ordre des birapports dans les séquences de birapports respectives suivant une règle prédéfinie commune à toutes les séquences de birapports, les séquences de birapports étant toutes différentes.

2. Mire de calibration selon la revendication précédente, dans laquelle tous les marqueurs (10) présentent sensiblement la même taille et sensiblement la même forme.

3. Mire de calibration selon l'une quelconque des revendications précédentes, dans laquelle les droites sont des génératrices, dites génératrices remarquables, d'un cylindre.

4. Mire de calibration selon l'une quelconque des revendications précédentes, dans laquelle la répartition des droites selon lesquelles les sous-ensembles de marqueurs sont alignés est choisie de façon que, pour toutes les conditions de projection dans lesquelles on acquiert des images lors de la calibration aucune projection de marqueur ne chevauche une autre projection de marqueur et/ou de façon que si des projections de marqueurs issues de deux sous-ensembles de marqueurs se chevauchent, les projections des marqueurs des autres sous-ensembles de marqueurs ne se chevauchent pas.

5. Mire de calibration selon l'une quelconque des revendications précédentes, dans laquelle les sous-ensembles de marqueurs sont répartis selon des segments droites remarquables respectifs parallèles à un axe z , les segments de droite étant de même longueur et présentant les même coordonnées selon ledit axe z, chaque segment de droite hébergeant un premier nombre m entier de sites ($S_{ij}$), susceptibles d'être occupés par un marqueur (10), deux sites consécutifs quelconques pris le long dudit segment remarquable ($g_i$) étant espacés d'une distance prédéterminée appelée pas (p), chaque site ($S_{ij}$) étant affecté d'une première valeur ou respectivement d'une deuxième valeur selon qu'il est occupé par un marqueur ou non, les marqueurs étant répartis de façon que les valeurs attribuées à un deuxième nombre entier n, au plus égal à m, de sites consécutifs quelconques pris selon un sens donné le long des segments de droites respectifs ($g_i$) forment des codes binaires respectifs composés de n bits, chaque code binaire composé de n bits formé selon ledit sens étant unique.

6. Mire de calibration selon la revendication précédente, dans laquelle le deuxième nombre m est supérieur au premier nombre n.

7. Mire de calibration selon l'une quelconque des revendications 5 à 6, dans laquelle les marqueurs sont répartis sur la mire de façon que, pour un nombre de génératrices et de sites par génératrice connus et pour des taux d'occupation connus des sites des génératrices respectives, une différence entre des codes binaires formés par les valeurs prises par les m sites consécutifs hébergés par les génératrices remarquables respectives selon le sens prédéfini est maximale, les codes binaires sont des portions d'une suite obtenue au moyen d'un LFSR de n bits, m étant inférieur ou égal à n.

8. Mire de calibration selon l'une quelconque des revendications précédentes dans laquelle l'ordre des birapports dans chaque séquence de birapports construite à partir d'un sous-ensemble de marqueurs alignés selon une droite, est défini de la façon suivante :

Pour des marqueurs notés $A_g$ portent les numéros d'ordre g = 1 à N le long de la droite selon un deuxième sens,

Pour i allant de 1 à N-3 faire :

(-Pour j allant de i+1 à N-2 faire :

(-Pour k allant de j+1 à N-1 faire :

(-Pour l allant de k+1 à N faire :

(-insérer le birapport suivant dans la séquence, le birapport suivant étant un birapport calculé à partir des marqueurs $A_{g=i}$, $A_{g=j}$, $A_{g=k}$, $A_{g=l}$,

- l=l+1)

- k=k+1)

- j=j+1),

-i=i+1),

Où N est un nombre entier.

**9.** Mire de calibration selon l'une quelconque des revendications précédentes dans laquelle l'ordre des marqueurs dans chaque quadruplet de marqueurs est l'ordre des marqueurs le long de la droite selon laquelle ils sont alignés selon le premier sens.

**10.** Procédé de détermination de caractéristiques géométriques d'un dispositif d'imagerie par rayons X destiné à réaliser des projections tridimensionnelles d'un objet par reconstruction à partir de projections bidimensionnelles dudit objet, ledit procédé utilisant une mire de calibration selon l'une quelconque des revendications précédentes, le procédé selon l'invention comprend les étapes suivantes :

- Placer (100) la mire de calibration dans une zone de projection entre une source (3) de rayons X et un détecteur (4) de rayons X,
- Acquérir (110) au moins une projection de la mire de calibration selon au moins une géométrie du dispositif d'imagerie définie par des positions de la source, de la mire et du détecteur et de leurs orientations relatives,

Et, pour chaque projection de la mire :

◦ Détecter (111) les projections de marqueurs dans la projection,
◦ Déterminer (112) les positions des projections de marqueurs dans la projection,
◦ Détecter (113) des alignements de projections des marqueurs selon des droites respectives appelées droites images,
Pour chaque alignement de projections de marqueurs :

◦ Former (114) une séquence dite séquence image de birapports à partir des projections de marqueurs formant ledit alignement, la séquence image comprenant un unique birapport par quadruplet de projections de marqueurs dans lequel les projections de marqueurs sont ordonnées selon l'ordre prédéfini dépendant des numéros d'ordre des projections de marqueurs respectives le long de la droite image correspondante selon le premier sens prédéfini, et , lorsqu'un ensemble de projections de marqueurs comprend au moins cinq projections marqueurs, l'ordre des birapports dans la séquences image suivant la règle prédéfinie le long de la droite image correspondante,
◦ Pour chaque projection de marqueur formant ledit alignement détectée, identifier (115) le marqueur dont elle est issue en attribuant une séquence de birapports formée à partir des projections de marqueur dudit alignement à une partie d'une séquence de birapports formée par des marqueurs de la mire comprenant le même nombre de birapports que la séquence image,

◦ Déterminer (116) des caractéristiques géométriques du dispositif d'imagerie lors de l'acquisition de la projection de la mire à partir des positions des projections de marqueurs détectées et des positions des marqueurs correspondants respectifs.

11. Procédé de calibration selon la revendication précédente, dans lequel l'étape de détection des alignements est réalisée en appliquant une transformée de Hough aux positions des projections marqueurs détectées lors de l'étape de détection des projections des marqueurs.

12. Procédé de fabrication d'une mire de calibration selon l'une quelconque des revendications 5 à 9, dans lequel les codes binaires sont des portions d'une suite obtenue au moyen d'un LFSR de n bits.

## Patentansprüche

1. Kalibrierungstestbild (1), das zum geometrischen Kalibrieren einer bildgebenden Vorrichtung durch Röntgenstrahlen bestimmt ist, die dazu bestimmt ist, dreidimensionale Bilder eines Objekts durch Rekonstruktion anhand von zweidimensionalen Projektionen des Objekts zu erzeugen, wobei das Kalibrierungstestbild (1) einen räumlichen Träger (20) umfasst, der über Marker (10) mit radiologischer Absorption verfügt, die in Bezug auf den räumlichen Träger (20) kontrastreich sind, wobei die Marker (10) gemäß einer dreidimensionalen Figur verteilt sind, **dadurch gekennzeichnet, dass** die Marker (10) in Marker-Untereinheiten verteilt sind, die gemäß jeweiliger im Wesentlichen paralleler Geraden so verteilt sind, dass die Doppelverhältnis-Sequenzen anhand von jeweiligen Marker-Untereinheiten konstruiert werden können, wobei jede Doppelverhältnis-Sequenz ein einmaliges Doppelverhältnis für jede Marker-Vierergruppe umfasst, in der die Marker gemäß einer Ordnung geordnet sind, die von den Ordnungsnummern der eweiligen Marker entlang der Geraden, gemäß der sie gemäß einer ersten vordefinierten Richtung ausgerichtet sind, abhängt, wobei die Ordnung für alle Doppelverhältnisse gleich ist, und wenn eine Marker-Untereinheit mindestens fünf Marker umfasst, die Ordnung der Doppelverhältnisse in den jeweiligen Doppelverhältnis-Sequenzen einer vordefinierten gemeinsamen Regel folgt für alle Doppelverhältnis-Sequenzen, wobei die Doppelverhältnis-Sequenzen alle verschieden sind.

2. Kalibrierungstestbild nach dem vorherigen Anspruch, wobei alle Marker (10) im Wesentlichen die gleiche Größe und im Wesentlichen die gleiche Form aufweisen.

3. Kalibrierungstestbild nach einem der vorherigen Ansprüche, wobei die Geraden Mantellinien, genannt erkennbare Mantellinien, eines Zylinders sind.

4. Kalibrierungstestbild nach einem der vorherigen Ansprüche, wobei die Verteilung der Geraden, nach denen die Marker-Untereinheiten ausgerichtet werden, so gewählt ist, dass für alle Projektionsbedingungen, unter denen bei der Kalibrierung Bilder erfasst werden, keine Marker-Projektion eine andere Marker-Projektion überlappt und/oder so dass wenn Marker-Projektionen, die von zwei Marker-Untereinheiten ausgegeben werden, sich überlappen, sich die Marker-Projektionen der anderen Marker-Untereinheiten nicht überlappen.

5. Kalibrierungstestbild nach einem der vorherigen Ansprüche, wobei die Marker-Untereinheiten gemäß erkennbaren Geradensegmenten verteilt sind, die jeweils parallel zu einer Z-Achse sind, wobei die Geradensegmente dieselbe Länge haben und dieselben Koordinaten gemäß der Z-Achse aufweisen, wobei jedes Geradensegment eine erste Ganzzahl m von Stellen (Sij) beherbergt, die von einem Marker (10) belegt werden können, wobei zwei aufeinanderfolgende beliebige Stellen entlang des erkennbaren Segments (gi) mit einem vorbestimmten Abstand beabstandet sind, der als Abstand Teilung (p) bezeichnet wird, wobei jede Stelle (Sij) einen ersten Wert bzw. einen zweiten Wert aufweist, je nachdem, ob sie von einem Marker belegt ist, wobei die Marker so verteilt sind, dass die zu einer zweiten Ganzzahl n zugewiesenen Werte, höchstens gleich m, von beliebigen aufeinanderfolgenden Stellen gemäß einer gegebenen Richtung entlang jeweiliger Geradensegmente (gi) jeweilige Binärcodes bilden, die aus n Bits bestehen, wobei jeder Binärcode, der aus n Bits besteht, die gemäß der Richtung gebildet sind, einmalig ist.

6. Kalibrierungstestbild nach dem vorherigen Anspruch, wobei die zweite Anzahl m größer als die erste Anzahl n ist.

7. Kalibrierungstestbild nach einem der Ansprüche 5 bis 6, wobei die Marker auf dem Testbild so verteilt sind, dass für eine Anzahl von bekannten Mantellinien und Stellen pro Mantellinie und für bekannte Belegungsraten von Stellen jeweiliger Mantellinien, eine Differenz zwischen den Binärcodes, gebildet durch die Werte, die durch die aufeinanderfolgenden m Stellen angenommen werden, die von den jeweiligen erkennbaren Mantellinien gemäß der vordefinierten Richtung beherbergt sind, maximal ist, wobei die Binärcodes Abschnitte einer Folge sind, die mittels eines LFSR mit n Bits erlangt wird, wobei n kleiner als oder gleich n ist.

8. Kalibrierungstestbild nach einem der vorherigen Ansprüche, wobei die Ordnung der Doppelverhältnisse in jeder Doppelverhältnis-Sequenz, die anhand einer Marker-Untereinheit, die gemäß einer Geraden ausgerichtet sind, aufgebaut ist, wie folgt definiert ist:

Für die Marker mit der Bezeichnung $A_g$, die die Ordnungsnummern $g = 1$ bis $N$ entlang der Geraden gemäß einer zweiten Richtung,

Für i, das von 1 bis N-3 reicht, machen:

(-Für j, das von i+1 bis N-2 reicht, machen:

(-Für k, das von j+1 bis N-1 reicht, machen:

(-Für l, das von k+1 bis N reicht, machen:

(-Einfügen des folgenden Doppelverhältnisses in die Sequenz, wobei das folgende Doppelverhältnis ein Doppelverhältnis ist, das berechnet wurde, anhand von den Markern

$A_{g=i}$, $A_{g=j}$, $A_{g=k}$, $A_{g=l}$,

- l=l+1)

- k=k+1)

-j=j+1).

-i=i+1),

wobei N eine Ganzzahl ist.

9. Kalibrierungstestbild nach einem der vorherigen Ansprüche, wobei die Ordnung der Marker in jeder Marker-Vierergruppe die Ordnung der Marker entlang der Geraden ist, gemäß der sie gemäß der ersten Richtung ausgerichtet sind.

10. Verfahren zum Bestimmen geometrischer Merkmale einer bildgebenden Vorrichtung durch Röntgenstrahlen, die dazu bestimmt ist, dreidimensionale Projektionen eines Objekts durch Rekonstruktion anhand von zweidimensionalen Projektionen des Objekts durchzuführen, wobei das Verfahren ein Kalibrierungstestbild nach einem der vorherigen Ansprüche verwendet, wobei das Verfahren gemäß der Erfindung die folgenden Schritte umfasst:

- Anbringen (100) des Kalibrierungstestbilds in einem Projektionsbereich zwischen einer Quelle (3) von Röntgenstrahlen und einem Detektor (4) von Röntgenstrahlen,
- Aufnehmen (110) von mindestens einer Projektion des Kalibrierungstestbilds gemäß mindestens einer Geometrie der bildgebenden Vorrichtung definiert durch die Positionen der Quelle, des Testbilds und des Detektors und ihrer relativen Ausrichtungen,

und für jede Projektion des Testbilds:

◦ Erfassen (111) der Marker-Projektionen in der Projektion,
◦ Bestimmen (112) der Positionen der Marker-Projektionen in der Projektion,
◦ Erfassen (113) der Ausrichtungen der Marker-Projektionen gemäß jeweiliger Geraden, genannt Bildgeraden,

Für jede Ausrichtung von Marker-Projektionen:

◦ Bilden (114) einer Sequenz genannten Doppelverhältnis-Bildsequenz anhand von Marker-Projektionen, die diese Ausrichtung bilden, wobei die Bildsequenz ein einmaliges Doppelverhältnis pro Vierergruppe von Marker-Projektionen umfasst, in dem die Marker-Projektionen gemäß der vordefinierten Ordnung abhängig von den Ordnungsnummern der jeweiligen Marker-Projektionen entlang der entsprechenden Bildgerade gemäß der ersten vordefinierten Richtung geordnet sind, und wenn eine Einheit von Marker-Projektionen mindestens fünf Marker-Projektionen umfasst, die Ordnung von Doppelverhältnissen in der Bildsequenz der vordefinierten Regel entlang der entsprechenden Bildgeraden folgt,

∘ Für jede Marker-Projektion, die die erfasste Ausrichtung bildet, Identifizieren (115) des Markers, von dem sie ausgegeben wird, unter Zuweisung einer Doppelverhältnis-Sequenz, die anhand von Marker-Projektionen der Ausrichtung gebildet wird, zu einem Teil einer Doppelverhältnis-Sequenz, die durch die Marker des Testbilds gebildet wird, umfassend die gleiche Anzahl von Doppelverhältnissen wie die Bildsequenz,

∘ Bestimmen (116) der geometrischen Merkmale der bildgebenden Vorrichtung bei der Aufnahme der Projektion des Testbilds anhand der Positionen der erfassten Marker-Projektionen und der Positionen der jeweils entsprechenden Marker.

**11.** Kalibrierungsverfahren nach dem vorherigen Anspruch, wobei der Erfassungsschritt der Ausrichtungen unter Anwendung einer Hough-Transformierten auf die Positionen der erfassten Marker-Projektionen beim Erfassungsschritt der Marker-Projektionen.

**12.** Herstellungsverfahren eines Kalibrierungstestbilds nach einem der Ansprüche 5 bis 9, wobei die Binärcodes Abschnitte einer Folge sind, die mittels einer LFSR mit n Bits erlangt wird.

## Claims

**1.** A calibration test pattern (1) intended to geometrically calibrate an X-ray imaging device intended to generate three-dimensional images of an object through reconstruction that is based on two-dimensional projections of said object, said calibration test pattern (1) comprising a volume support (20) provided with markers (10) with radiological absorption that is contrasted relative to said volume support (20), said markers (10) being distributed according to a three-dimensional pattern, **characterised in that** said markers (10) are distributed in sub-sets of markers distributed along substantially parallel respective straight lines so that sequences of cross-ratios can be constructed from the sub-sets of respective markers, each sequence of cross-ratios comprising a single cross-ratio for each quadruplet of markers in which the markers are ordered according to an order that depends on the order numbers of the respective markers along the straight line on which they are aligned in a first predetermined direction, said order being common to all the cross-ratios, and when a sub-set of markers comprises at least five markers, the order of the cross-ratios in the sequences of respective cross-ratios follows a predetermined rule that is common to all the sequences of cross-ratios, the sequences of cross-ratios all being different.

**2.** The calibration test pattern according to the preceding claim, wherein all the markers (10) have substantially the same size and substantially the same shape.

**3.** The calibration test pattern according to any one of the preceding claims, wherein the straight lines are generating lines, called observable generating lines, of a cylinder.

**4.** The calibration test pattern according to any one of the preceding claims, wherein the distribution of the straight lines along which the sub-sets of markers are aligned is selected so that, for all the projection conditions in which images are acquired during the calibration, no marker projection overlaps another marker projection and/or so that if marker projections originating from two sub-sets of markers overlap, the projections of the markers of the other sub-sets of markers do not overlap.

**5.** The calibration test pattern according to any one of the preceding claims, wherein the sub-sets of markers are distributed along respective observable straight line segments parallel to an axis z, the straight line segments being the same length and having the same coordinates along said axis z, each straight line segment accommodating a first integer m of sites ($S_{ij}$) likely to be occupied by a marker (10), any two consecutive sites taken along said observable segment ($g_i$) being spaced apart by a predetermined distance called step (p), each site ($S_{ij}$) being assigned a first value or respectively a second value depending on whether or not it is occupied by a marker, the markers being distributed so that the values assigned to a second integer n, at most equal to m, of any consecutive sites taken in a given direction along respective straight line segments ($g_i$) form respective binary codes made up of n bits, with each binary code made up of n bits formed along said direction being unique.

**6.** The calibration test pattern according to the preceding claim, wherein the second number m is greater than the first number n.

**7.** The calibration test pattern according to any one of claims 5 to 6, wherein the markers are distributed on the pattern so that, for a known number of generating lines and sites per generating line and for known occupancy rates of the sites of the respective generating lines, a difference between the binary codes formed by the values taken by the m consecutive sites accommodated by the respective observable generating lines along the predefined direction is maximal, the binary codes being portions of a series obtained by means of an LFSR of n bits, with m being less than or equal to n.

**8.** The calibration test pattern according to any one of the preceding claims, wherein the order of the cross-ratios in each sequence of cross-ratios constructed from a sub-set of markers aligned along a straight line is defined as follows:

for markers denoted $A_g$ having order numbers g = 1 to N along the straight line in a second direction,

for i ranging from 1 to N-3 then:
(-for j ranging from i+1 to N-2 then:
(-for k ranging from j+1 to N-1 then:
(-for l ranging from k+1 to N then:
(-insert the next cross-ratio in the sequence, the next cross-ratio being a cross-ratio computed on the basis of the markers $A_{g=i}$, $A_{g=j}$, $A_{g=k}$, $A_{g=l}$,
- l=l+1),
- k=k+1),
- j=j+1),
-i=i+1),

where N is an integer.

**9.** The calibration test pattern according to any one of the preceding claims, wherein the order of the markers in each quadruplet of markers is the order of the markers along the straight line on which they are aligned in the first direction.

**10.** A method for determining geometrical characteristics of an X-ray imaging device intended to generate three-dimensional projections of an object through reconstruction that is based on two-dimensional projections of said object, said method using a calibration test pattern according to any one of the preceding claims, said method according to the invention comprising the following steps:

- placing (100) the calibration test pattern in a projection zone between an X-ray source (3) and an X-ray detector (4);
- acquiring (110) at least one projection of the calibration test pattern according to at least one geometry of said imaging device that is defined by positions of said source, said test pattern and said detector and their relative orientations;

and, for each projection of said test pattern:

o detecting (111) the projections of markers in the projection;
o determining (112) the positions of the projections of markers in the projection;
o detecting (113) alignments of projections of the markers in the respective straight lines called image straight lines,

for each alignment of projections of markers:

o forming (114) a sequence, called cross-ratio image sequence, on the basis of the projections of markers forming said alignment, the image sequence comprising a single cross-ratio per quadruplet of projections of markers, in which the projections of markers are ordered according to the predetermined order depending on the order numbers of the projections of respective markers along the corresponding image straight line in the first predetermined direction, and, when a set of projections of markers comprises at least five marker projections, the order of the cross-ratios in the image sequence follows the predetermined rule along the corresponding image straight line;

o identifying (115), for each marker projection forming said detected alignment, the marker from which it originates by assigning a sequence of cross-ratios formed on the basis of the marker projections of said alignment to a section of a cross-ratio sequence formed by the markers of the test pattern comprising the same number of cross-ratios as the image sequence;

o determining (116) geometrical characteristics of the imaging device during the acquisition of the projection of the test pattern on the basis of the positions of the projections of detected markers and the positions of the respective corresponding markers.

11. The calibration method according to the preceding claim, wherein the step of detecting alignments is carried out by applying a Hough transform to the positions of the marker projections detected during the step of detecting marker projections.

12. A method for generating a calibration test pattern according to any one of claims 5 to 9, wherein the binary codes are portions of a series obtained by means of an LFSR of n bits.

FIG.1

FIG.2

FIG.3a          FIG.3b

Placer la mire — 100

Acquérir au moins une projection de la mire — 110

Pour chaque projection de la mire

Détecter les projections des marqueurs — 111

Positionner les projections des marqueurs — 112

Détecter les alignements de marqueurs — 113

Pour chaque alignement

Former une séquence image — 114

Attribuer un marqueur à chaque projection de marqueur — 115

Déterminer des caractéristiques géométriques du dispositif d'imagerie lors de l'acquisition de la projection de la mire — 116

FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6715918 B